# EUROPEAN PATENT SPECIFICATION

(11) **EP 0 565 594 B1**
(45) Date of publication and mention of the grant of the patent: **07.06.1995**
(21) Application number: 92902939.5
(22) Date of filing: 06.01.1992
(51) Int. Cl.: G01N 33/53

(54) **APPARATUS FOR DRY CHEMICAL ANALYSIS OF FLUIDS**
VORRICHTUNG ZUR TROCKENEN CHEMISCHEN ANALYSE VON FLÜSSIGKEITEN
APPAREIL SERVANT A EFFECTUER UNE ANALYSE CHIMIQUE SECHE DE FLUIDES

(30) Priority: 06.01.1991 IL 96887
(43) Date of publication of application: 20.10.1993
(73) Proprietor: ORGENICS LTD., Yavne 70 650 (IL)
(72) Inventor: FISH, Falk, 69 497 Tel Aviv (IL)
(74) Representative: de Bruijn, Leendert C.
(86) International application number: NL9200002
(87) International publication number: WO9212425

(56) References cited:
- EP-A- 269 876
- EP-A- 336 483
- US-A- 4 912 034

## Description

### FIELD OF THE INVENTION

The invention relates to apparatus for dry chemical analysis of fluids.

### BACKGROUND OF THE INVENTION

Test devices for dry chemical analysis of fluids typically include one or more reagent matrix areas retained in a holder or attached to a substrate. These reagent matrix areas detect chemical components of sample liquids by a color reaction formed when the reagent contacts the liquid sample.

Test devices or strips for dry chemical analysis of fluids are well established in the art and used in agriculture, industry and medicine. For these applications it is desirable that the test devices be simple and inexpensive to produce.

Such devices are described in U.S. Patents 4,774,054, 3,798,004 and 4,647,430 in which multilayer filters are glued to a substrate. The use of glue, however, can have a detrimental effect on the reagents used and their storage life as well as complicating the assembly of the device.

The use of a filter holder in a test device , such as in U.S. Patent 4,912,034, eliminates the need for the use of glue in the test device, but also increases the device's complexity.

A simplified filter holder test device is described in U.S. Patent 2,785,057, however this device employs a perforated disk filter retaining member abutting an absorbent layer which would not be suitable for chemical analysis of whole blood samples.

Accordingly there is a need in the art for a simple device for dry chemical analysis of fluids which can be used for chemical analysis of whole blood samples. The present invention fulfills this need and provides related advantages.

### SUMMARY OF THE INVENTION

There is thus provided in accordance with the present invention apparatus for dry chemical analysis of fluids comprising a filter, a filter holder apparatus including a base member defining a filter supporting location and a filter retaining apparatus including a mesh arranged to retain the filter at the filter supporting location in spaced relationship with respect to the mesh.

In accordance with a preferred embodiment of the invention the base member is generally rigid.

In accordance with another preferred embodiment of the invention the filter supporting location includes a depression.

In accordance with yet another preferred embodiment of the invention the base member is elongated and comprises first and second ends and wherein the depression in the base member is located at the first end.

In accordance with still another preferred embodiment of the invention the filter retaining apparatus abuts the base member and the base member and the filter retaining apparatus are mechanically joined.

In accordance with a further preferred embodiment of the invention the filter retaining apparatus has an outer side surface and the base member has an inner side surface which encloses the side surface of the filter retaining apparatus.

In accordance with yet a further preferred embodiment of the invention a portion of the side surface of the filter retaining apparatus is formed with flange and a portion of the side surface of the base member is formed with a notch and wherein the flange engages the notch to mechanically join the filter retaining apparatus to the base member.

In accordance with still a further preferred embodiment of the invention the filter retaining apparatus additionally includes an outer solid portion surrounding the mesh.

In accordance with a preferred embodiment of the invention the filter retaining apparatus additionally includes protuberances depending perpendicularly from the mesh and wherein the protuberances abut the filter for retaining the filter within the depression and for providing even distribution of a sample liquid on a surface of the filter.

In accordance with another preferred embodiment of the invention the filter retaining apparatus additionally includes protuberances depending perpendicularly from a part of the solid portion adjacent the mesh and wherein the protuberances abut the filter for retaining the filter within the depression.

In accordance with still another preferred embodiment of the invention the filter retaining apparatus additionally includes a flange depending perpendicularly from the solid portion adjacent the mesh and wherein the flange is spaced from the filter for increasing the rate of flow of sample liquid to the filter and for reducing the seepage of sample liquid from the depression along an interface between the base member and the solid portion of the filter retaining apparatus.

In accordance with yet another preferred embodiment of the invention the filter holder apparatus is fabricated from a hydrophilic material.

In accordance with a further preferred embodiment of the invention at least one surface of the base member is treated to induce hydrophilousity.

In accordance with a still further preferred embodiment of the invention at least one surface of the filter retaining apparatus is treated to induce hydrophilousity.

In accordance with yet a further preferred embodiment of the invention the filter retaining apparatus is formed from an injection moldable plastic.

In accordance with another preferred embodiment of the invention the base member is formed from an injection moldable plastic.

In accordance with still another preferred embodiment of the invention the filter holder apparatus does not exceed 5 cm in length, 2 cm in width and 0.5 cm in depth.

In accordance with yet another preferred embodiment of the invention the base member includes an aperture disposed between the depression and a bottom surface of the base member to permit fluid communication between the depression and the bottom surface of the base member.

In accordance with a further preferred embodiment of the invention the filter is a multilayer filter.

In accordance with a still further preferred embodiment of the invention one layer of the multilayer filter includes a reagent impregnated material.

### BRIEF DESCRIPTION OF THE DRAWINGS

The present invention will be understood and appreciated more fully from the following detailed description taken in conjunction with the drawings in which:
Fig. 1A is a schematic top view illustration of apparatus for dry chemical analysis of fluids constructed and operative in accordance with a preferred embodiment of the invention;
Fig. 1B is a schematic view illustration of the unassembled apparatus of Fig. 1A;
Fig. 1C is a schematic bottom view of the apparatus of Fig. 1A;
Fig. 2A is a schematic top view illustration of a filter retaining apparatus in an alternative embodiment of the apparatus of Fig. 1A;
Fig. 2B is a schematic top view of the top surface of the base member in an alternative embodiment of the apparatus of Fig. 1B;
Fig. 2C is a schematic bottom view illustration of the bottom surface of the base member in an alternative embodiment of the apparatus of Fig. 1C;
Fig. 3 is a schematic side sectional view illustration of the apparatus of Fig. 1;
Fig. 4A is an exploded schematic side sectional view illustration of the apparatus of Fig. 1;
Fig. 4B is an exploded schematic side sectional view of an alternative embodiment of the apparatus of Fig 4A; and
Fig. 5 is a schematic side sectional view of the apparatus of Fig 2.

### DETAILED DESCRIPTION OF PREFERRED EMBODIMENTS

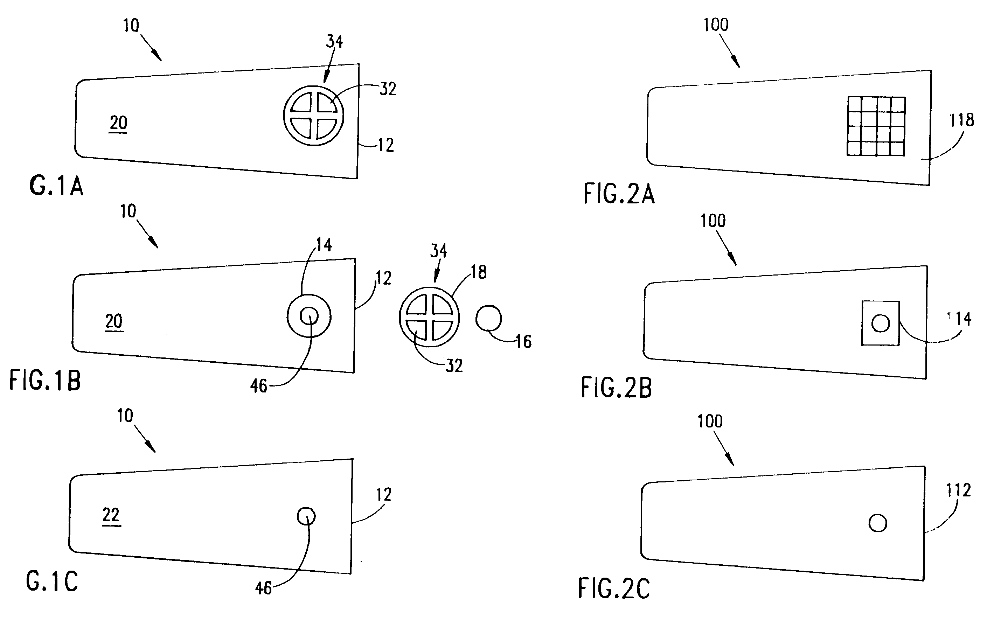

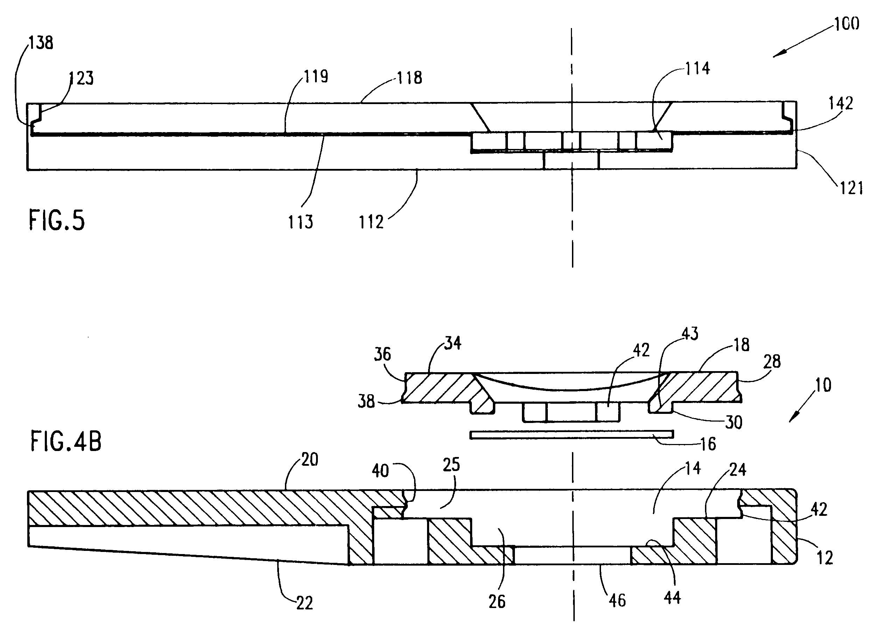

Reference is now made to Figs. 1A, 1B, 1C, 3 and 4A which illustrate apparatus for dry chemical analysis of fluids constructed and operative in accordance with a preferred embodiment of the present invention and comprising a generally elongated shaped filter holder 10 having a base member 12 approximately 5cm long, 2 cm wide and 0.5 cm thick with a top surface 20 and a bottom surface 22.

One end of the base member 12 has a typically circular depression 14 extending from top surface 20 toward bottom surface 22. The diameter of depression 14 is approximately 1.2 cm at the level of top surface of 20. At a depth of 0.10 cm depression 14 sharply narrows to a diameter of 0.70 cm forming a ledge 24. Ledge 24 separates upper portion 25 of depression 14 from a lower portion 26. At the level of ledge 24 depression 14 has a wall in which a notch 40 is formed. Lower portion 26 has a base 44 which is approximately 0.20 cm from top surface 20 and 0.1 cm from ledge 24 and from which a bore 46 extends to the bottom surface 22 of base member 12.

Filter 16 is disposed in the lower portion 26 of depression 14. Filter 16 is typically 0.1 mm - 1.0 mm thick and has approximately the same diameter as the lower portion 26 of depression 14.

Filter 16 is retained in depression 14 by filter retaining apparatus 18. Filter retaining apparatus 18 has a solid portion 34 surrounding an irregular mesh surface portion 32. Irregular mesh surface portion 32 has an average mesh opening of approximately 2 - 4 mm.

Filter retaining apparatus 18 additionally has an upper portion 28 and a lower portion 30. The solid portion 34 of filter retaining apparatus 18 has a side surface from which a flange 38 laterally extends. The lower portion 30 of filter retaining apparatus 18 includes protuberances 42 depending perpendicularly from the mesh surface portion 32 and from a part of the solid portion 34 of filter retaining apparatus 18.

Lower portion 30 of filter retaining apparatus 18 is sized to fit tightly into lower portion 26 of depression 14 with the protuberances 42 abutting filter 16. Upper portion 28 of filter retaining apparatus 18 is sized to fit tightly into upper portion 25 of depression 14. The solid portion 34 of filter retaining apparatus 18 rests on ledge 24 and flange 38 engages notch 40 to mechanically join filter retaining apparatus 18 to base member 12. Filter 16 is thus securely retained in depression 14.

Filter 16 is typically a multilayer filter in which the top layer is a glass fiber filter and the bottom layer is a reagent impregnated nylon membrane.

Filter retaining apparatus 18 and base member 12 are typically fabricated from a plastic material which although generally rigid is flexible enough to permit flange 38 to flex during assembly of apparatus 10. During assembly of apparatus 10 filter 16 is first placed in depression 14. Retaining apparatus 18 is then inserted in depression 14 and gently pressed toward bottom surface 22 of base member 12. Flange 38 is deformed until solid portion 34 abuts ledge 24 releasing the pressure on flange 38. Flange 38 then returns to its original shape and engages notch 40.

Filter retaining apparatus 18 and base member 12 are typically fabricated by injection molding from ABS plastic. Other plastics, however, such as polystyrene or polypropylene may alternatively be used as well as other fabrication methods such as blow molding.

Typically, filter retaining apparatus 18 and base member 12 are treated to make their surfaces hydrophilic. This treatment typically includes first exposing filter retaining apparatus 18 and base member 12 to a corona discharge for approximately 1 - 5 seconds at a distance of 1mm from the electrode. For the filter retaining apparatus 18 the treatment additionally typically includes a brief complete immersion in a surfactant solution such as a 0.01 - 0.5M solution of dioctyl sulfosuccinate sodium salt dissolved in a 4:6 alcohol water mixture. Alternatively, filter retaining apparatus 18 and base member 12 may be fabricated from a hydrophilic material.

In using the apparatus 10 illustrated in Figs. 1A, 1B, 1C, 3 and 4 a sample liquid, typically whole blood, is placed on mesh surface portion 32 of filter retaining apparatus 18. The blood is generally evenly distributed in the space over the glass fiber filter layer by the mesh surface portion 32 and the protuberances 42.

The blood is then drawn through the glass fiber filter by gravity and capillary action. The glass fiber filter separates the formed portion of the blood, such as erythrocytes, from the liquid portion which is passed on to the reagent impregnated nylon membrane.

When the liquid portion of the blood contacts the nylon membrane, chemical components of the blood react with reagents in the nylon membrane to cause a color change in the reagents thus detecting the presence and/or concentration of these chemical components. This color change can be seen from the bottom surface 22 of base member 12 through a bore 46. Bore 46 also permits excess liquid to pass out of the lower portion of base member 12 to prevent backflow pressure, which would interfere with the flow of the sample liquid through the filter.

Reference is now made to Figs. 2A, 2B, 2C and 5 which illustrate an alternative preferred embodiment of the present invention. This embodiment of apparatus for dry chemical analysis of fluids (labeled 100 in the figures) differs from the embodiment of Figs 1A, 1B, 1C, 3 and 4A only in that filter retaining apparatus 118 and base member 112 have respective bottom surfaces 119 and top surfaces 113 which are congruent at all points except depression 114. Retaining member 118 and base member 112 are joined mechanically in apparatus similar to that illustrated in the embodiment of Figs. 1A, 1B, 1C, 3 and 5. Base member 112 has a perimeter surface 121 formed with a notch 142. Retaining member 118 has a perimeter surface 123 formed with a flange 138. When bottom surface 119 of filter retaining apparatus 118 abuts top surface 113 of base member 112, flange 138 engages notch 142 mechanically joining filter retaining apparatus 118 to base member 112.

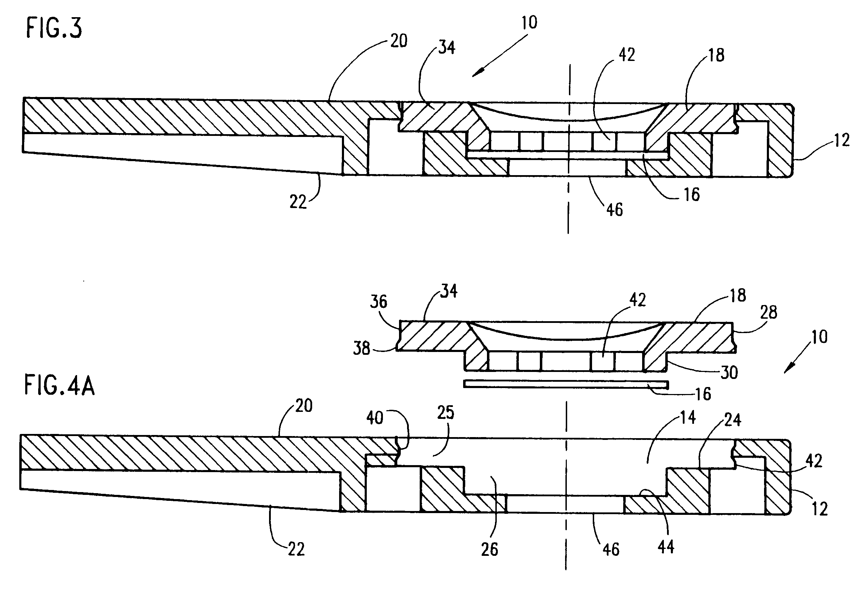

Reference is now made to Fig. 4B which illustrates an alternative embodiment of the invention in which a flange 43 depends perpendicularly from the solid portion of the filter retaining apparatus 18 and is spaced from filter 16. Flange 43 which is adjacent to and surrounding the mesh surface portion 32 is spaced from the filter 16 to increase the rate of flow of sample liquid to filter 16 and to reduce seepage of the sample liquid from depression 14 along the interface between base member 12 and the solid portion 34 of filter retaining apparatus 18.

## Claims

1. Apparatus for dry chemical analysis of fluids comprising:
a filter; and
filter holder means including:
a base member defining a filter supporting location; and
filter retaining means including a mesh arranged to retain said filter at said filter supporting location in spaced relationship with respect to said mesh.

2. Apparatus according to claim 1 wherein said base member is rigid.

3. Apparatus according to claim 1 wherein said filter supporting location includes a depression.

4. Apparatus according to claim 3 wherein said base member is elongated and comprises first and second ends and wherein said depression in said base member is located at said first end.

5. Apparatus according to any of the preceding claims wherein a portion of said filter retaining means abuts said base member and said base member and said filter retaining means are mechanically joined.

6. Apparatus according to any of the preceding claims wherein said filter retaining means has an outer side surface and said base member has an inner side surface which encloses said side surface of said filter retaining means.

7. Apparatus according to any of the preceding claims wherein a portion of said side surface of said filter retaining means is formed with a flange and a portion of said side surface of said base member is formed with a notch and wherein said flange engages said notch to mechanically join said filter retaining means to said base member.

8. Apparatus according to any of claims 1 - 6 wherein said filter retaining means additionally includes an outer solid portion surrounding said mesh.

9. Apparatus according to any of claims 3 - 8 wherein said filter retaining means additionally includes protuberances depending perpendicularly from said mesh and wherein said protuberances abut said filter for retaining said filter within said depression and for providing even distribution of a sample liquid on a surface of said filter.

10. Apparatus according to claims 8 and 9 wherein said filter retaining means additionally includes protuberances depending perpendicularly from a part of said solid portion adjacent said mesh and wherein said protuberances abut said filter for retaining said filter within said depression.

11. Apparatus according to claims 8 and 9 wherein said filter retaining means additionally includes a flange depending perpendicularly from said solid portion adjacent said mesh and wherein said flange is spaced from said filter for increasing the rate of flow of the sample liquid to said filter and for reducing seepage of the sample liquid from said depression along an interface between said base member and said solid portion of said filter retaining means.

12. Apparatus according to any of the preceding claims wherein said filter holder means is fabricated from a hydrophilic material.

13. Apparatus according to any of claims 1 - 7 or 12 wherein at least one surface of said base member is treated to induce hydrophilousity.

14. Apparatus according to any of claims 1 or 4 - 12 wherein at least one surface of said filter retaining means is treated to induce hydrophilousity.

15. Apparatus according to any of claims 1, 4 - 12 or 14 wherein said filter retaining means is formed from an injection moldable plastic.

16. Apparatus according any of claims 1 - 7, 12, or 13 wherein said base member is formed from an injection moldable plastic.

17. Apparatus according to claim 1 wherein said filter holder means does not exceed 5 cm in length, 2 cm in width and 0.5 cm in depth.

18. Apparatus according to any of claims 3 - 7 or 12 - 13 wherein said base member includes an aperture disposed between said depression and a bottom surface of said base member to permit fluid communication between said depression and said bottom surface of said base member.

19. Apparatus according to claim 1 wherein said filter is a multilayer filter.

20. Apparatus according to claim 19 wherein one layer of said multilayer filter includes a reagent impregnated material.

## Patentansprüche

1. Vorrichtung zur chemischen Trockenanalyse von Fluiden mit
- einem Filter; und
- einer Filterhalteeinrichtung, die einschließt ein Basisteil, das eine das Filter tragende Stelle definiert, und eine das Filter haltende Einrichtung, die ein Sieb einschließt, das in der Weise angeordnet ist, daß es das Filter an der das Filter tragenden Stelle in beabstandeter Beziehung bezüglich des Siebs hält.

2. Vorrichtung nach Anspruch 1, worin das Basisteil starr ist.

3. Vorrichtung nach Anspruch 1, worin die das Filter tragende Stelle eine Vertiefung einschliesst.

4. Vorrichtung nach Anspruch 3, worin das Basisteil länglich ist und ein erstes und ein zweites Ende umfaßt und worin der Unterdruckbereich in dem Basisteil an dem ersten Ende liegt.

5. Vorrichtung nach einem der vorangehenden Ansprüche, worin ein Abschnitt der das Filter haltenden Einrichtung an das Basisteil anstößt und das Basisteil und die das Filter haltende Einrichtung mechanisch verbunden sind.

6. Vorrichtung nach einem der vorangehenden Ansprüche, worin die das Filter haltende Einrichtung eine Außenseiten-Fläche aufweist und das Basisteil eine Innenseiten-Fläche aufweist, die die Seitenfläche der das Filter haltenden Einrichtung einschließt.

7. Vorrichtung nach einem der vorangehenden Ansprüche, worin ein Abschnitt der Seitenfläche der das Filter haltenden Einrichtung mit einem Flansch ausgebildet ist und ein Abschnitt der Seitenfläche des Basisteils mit einer Kerbe ausgebildet ist und worin der Flansch in die Kerbe eingreift und die das Filter haltende Einrichtung mit dem Basisteil mechanisch verbindet.

8. Vorrichtung nach einem der Ansprüche 1 bis 6, worin die das Filter haltende Einrichtung zusätzlich einen äußeren festen Abschnitt einschließt, der das Sieb umgibt.

9. Vorrichtung nach einem der Ansprüche 3 bis 8, worin die das Filter haltende Einrichtung zusätzlich Vorsprünge einschließt, die senkrecht von dem Sieb abstehen, und worin die Vorsprünge an das Filter anstoßen, um das Filter innerhalb der Unterdruckbereichs zu halten und um für eine gleichmäßige Verteilung einer Probenflüssigkeit auf einer Oberfläche des Filters zu sorgen.

10. Vorrichtung nach den Ansprüchen 8 und 9, worin die das Filter haltende Einrichtung zusätlich Vorsprünge einschließt, die senkrecht von einem Teil des festen Abschnitts, der dem Sieb benachbart ist, abstehen und worin die Vorsprünge an das Filter anstoßen, um das Filter innerhalb der Vertiefung zu halten.

11. Vorrichtung nach den Ansprüchen 8 und 9, worin die das Filter haltende Einrichtung zusätzlich einen Flansch einschließt, der senkrecht von dem festen Abschnitt, der dem Sieb benachbart ist, absteht und worin der Flansch von dem Filter beabstandet ist, um die Strömungsgeschwindigkeit der Probenflüssigkeit zu dem Filter zu erhöhen und um ein Auslaufen der Probenflüssigkeit aus der Vertiefung entlang der Grenzfläche zwischen dem Basisteil und dem festen Abschnitt der das Filter haltenden Einrichtung zu vermindern.

12. Vorrichtung nach einem der vorangehenden Ansprüche, worin die Filterhalteeinrichtung aus einem hydrophilen Material hergestellt ist.

13. Vorrichtung nach einem der Ansprüche 1 bis 7 oder 12, worin wenigstens eine Oberfläche des Basisteils unter Induzieren von Hydrophilie behandelt ist.

14. Vorrichtung nach einem der Ansprüche 1 oder 4 bis 12, worin wenigstens eine Oberfläche der das Filter haltenden Einrichtung unter Induzieren von Hydrophilie behandelt ist.

15. Vorrichtung nach einem der Ansprüche 1, 4 bis 12 oder 14, worin die das Filter haltende Einrichtung aus einem durch Spritzgießen formbaren Kunststoff gebildet ist.

16. Vorrichtung nach einem der Ansprüche 1 bis 7, 12 oder 13, worin das Basisteil aus einem durch Spritzgießen formbaren Kunststoff gebildet ist.

17. Vorrichtung nach Anspruch 1, worin die Filterhalteeinrichtung eine Länge von 5 cm, eine Breite von 2 cm und eine Tiefe von 0,5 cm nicht überschreitet.

18. Vorrichtung nach einem der Ansprüche 3 bis 7 oder 12 bis 13, worin das Basisteil eine zwischen dem Unterdruckbereich und einer unteren Fläche des Basisteils angeordnete Öffnung einschließt, die eine Fluidverbindung zwischen der Vertiefung und der unteren Fläche des Basisteils erlaubt.

19. Vorrichtung nach Anspruch 1, worin das Filter ein Mehrschichtenfilter ist.

20. Vorrichtung nach Anspruch 19, worin eine Schicht des Mehrschichtenfilters ein mit einem Reagens imprägniertes Material einschließt.

## Revendications

1. Appareil pour l'analyse chimique sèche de fluides, comprenant :
un filtre ; et
un moyen de support du filtre incluant :
un élément de base définissant un emplacement de support du filtre ; et
un moyen de maintien du filtre incluant un treillis arrangé pour maintenir ledit filtre audit emplacement de support du filtre en relation espacée par rapport audit treillis.

2. Appareil selon la revendication 1, dans lequel ledit élément de base est rigide.

3. Appareil selon la revendication 1, dans lequel ledit emplacement de support du filtre inclut une dépression.

4. Appareil selon la revendication 3, dans lequel ledit élément de base est allongé et comprend des première et seconde extrémités, et dans lequel ladite dépression dans ledit élément de base est situé à ladite première extrémité.

5. Appareil selon l'une quelconque des revendications précédentes, dans lequel une partie dudit moyen de maintien du filtre bute contre ledit élément de base, et ledit élément de base et ledit moyen de maintien du filtre sont joints mécaniquement.

6. Appareil selon l'une quelconque des revendications précédentes, dans lequel ledit moyen de maintien du filtre a une surface latérale extérieure et ledit élément de base a une surface latérale intérieure qui inclut ladite surface latérale dudit moyen de maintien du filtre.

7. Appareil selon l'une quelconque des revendications précédentes, dans lequel une partie de ladite surface latérale dudit moyen de maintien du filtre est munie d'un épaulement, et une partie de ladite surface latérale dudit élément de base est munie d'une encoche, et dans lequel ledit épaulement s'engage avec ladite encoche pour joindre mécaniquement ledit moyen de maintien du filtre audit élément de base.

8. Appareil selon l'une quelconque des revendications 1 à 6, dans lequel ledit moyen de maintien de filtre inclut en plus une partie extérieure solide entourant ledit treillis.

9. Appareil selon l'une quelconque des revendications 3 à 8, dans lequel ledit moyen de maintien du filtre inclut en outre des protubérances dépendant verticalement dudit treillis, et dans lequel lesdites protubérances butent contre ledit filtre pour maintenir ledit filtre dans ladite dépression et pour obtenir une distribution régulière d'un échantillon liquide sur la surface dudit filtre.

10. Appareil selon les revendications 8 et 9, dans lequel ledit moyen de maintien du filtre inclut en plus des protubérances dépendant perpendiculairement d'une partie de ladite partie solide de façon adjacente audit treillis, et dans lequel lesdites protubérances butent contre ledit filtre pour maintenir ledit filtre dans ladite dépression.

11. Appareil selon les revendications 8 et 9, dans lequel ledit moyen de maintien du filtre inclut en plus un épaulement dépendant perpendiculairement de ladite partie solide de façon adjacente audit treillis, et dans lequel ledit épaulement est espacé dudit filtre pour accroître la vitesse de flux de l'échantillon liquide audit filtre et pour réduire une infiltration de l'échantillon liquide à partir de ladite dépression, le long une interface entre ledit élément de base et ladite partie solide dudit moyen de maintien du filtre.

12. Appareil selon l'une quelconque des revendications précédentes, dans lequel ledit moyen de support du filtre est fabriqué à partir d'un matériau hydrophile.

13. Appareil selon l'une quelconque des revendications 1 à 7, ou 12, dans lequel au moins une surface dudit élément de base est traitée pour conférer une hydrophilie.

14. Appareil selon l'une quelconque des revendications 1, ou 4 à 12, dans lequel au moins une surface dudit moyen de maintien du filtre est traité pour conférer une hydrophilie.

15. Appareil selon l'une quelconque des revendications 1, 4 à 12, ou 14, dans lequel ledit moyen de maintien du filtre est formé à partir d'une matière plastique moulable par injection.

16. Appareil selon l'une quelconque des revendications 1 à 7, 12 ou 13, dans lequel ledit élément de base est formé à partir d'une matière plastique moulable par injection.

17. Appareil selon la revendication 1, dans lequel ledit moyen de support du filtre ne dépasse pas 5 cm de longueur, 2 cm de largeur et 0,5 cm de profondeur.

18. Appareil selon l'une quelconque des revendications 3 à 7, ou 12 et 13, dans lequel ledit élément de base inclut une ouverture disposée entre ladite dépression et la surface inférieure dudit élément de base pour permettre une communication fluide entre ladite dépression et la surface inférieure dudit élément de base.

19. Appareil selon la revendication 1, dans lequel ledit filtre est un filtre à couches multiples.

20. Appareil selon la revendication 19, dans lequel une couche dudit filtre à couches multiples inclut un matériau imprégné de réactif.
